# EUROPEAN PATENT SPECIFICATION

(11) **EP 3 603 550 B1**
(45) Date of publication and mention of the grant of the patent: **23.10.2024**
(21) Application number: 18772158.4
(22) Date of filing: 23.03.2018
(51) Int. Cl.: A61M 29/00, A61B 17/34, A61B 90/00

(54) **DILATOR**
DILATATOR
DILATATEUR

(30) Priority: 24.03.2017 WO PCT/JP2017/012024
(43) Date of publication of application: 05.02.2020
(73) Proprietor: Asahi Intecc Co., Ltd., Seto-shi, Aichi 489-0071 (JP)
(72) Inventor: FUSEYA, Yukihiro, Seto-shi, Aichi 489-0071 (JP); MAKI, Hideaki, Seto-shi, Aichi 489-0071 (JP); TAKAHASHI, Daiki, Seto-shi, Aichi 489-0071 (JP); SAWAI, Akira, Seto-shi, Aichi 489-0071 (JP); KITAI, Marina, Seto-shi, Aichi 489-0071 (JP)
(74) Representative: Winter, Brandl - Partnerschaft mbB
(86) International application number: PCT/JP2018/011672
(87) International publication number: WO 2018/174241

(56) References cited:
- WO-A1-91/07202
- DE-A1- 19 636 318
- JP-A- 2002 177 289
- JP-A- 2007 098 120
- JP-A- 2016 013 285
- US-A- 5 630 813
- US-A1- 2002 077 655
- US-A1- 2011 098 531
- US-A1- 2011 213 316
- US-A1- 2014 046 357
- US-A1- 2014 046 357

## Description

### TECHNICAL FIELD

The present invention relates to a dilator.

### BACKGROUND ART

Dilators are known for expanding a hole formed on the wall of the patient's digestive tract and the like for the purpose of treatment. A front end of a dilator is inserted into the hole formed on a wall, and a tapered portion is then pushed into the hole to expand the hole. Such dilators are disclosed in, for example, JP 2008-11867 A, US 2014/046357 A1, US 2002/077655 A1, US 5630813 A, US 2011/213316 A1, DE 19636318 A1, US 2011/098531 A1, and WO 91/07202 A1.

Specifically, US 2014/046357 A1 discloses a dilator with projections protruding from an outer peripheral surface of a tapered front end tip of a hollow shaft, wherein the projections are arranged in a spiral pattern and with a pitch increasing from a proximal end to a distal end of the distal tip.

Further, US 2002/077655 A1 discloses a dilator with a thread protruding from an outer peripheral surface of a tapered front end tip of a hollow shaft, wherein the thread may have different thread pitches and be formed such that it cuts automatically.

Further, US 5630813 A discloses a dilator in form of a tapered skin seal. The skin seal has single-lead helical thread or dual-lead helical threads protruding from its outer peripheral surface, wherein an inclination angle is shown to decrease toward a distal end of the skin seal.

### SUMMARY OF THE INVENTION

### TECHNICAL PROBLEMS

When the dilator described above is very long, a rotational force and a pushing force from the hand side may not be well transmitted to the front end of the dilator, and thus a hole formed on the wall of the digestive tract cannot be sufficiently expanded.

Starting from 2008-11867 A, US 2002/077655, or US 5630813 A, an object of the present invention is to provide a dilator with a hollow shaft having a spirally-arranged protruding portion which can be easily formed.

### SOLUTION TO PROBLEM

The above object is solved by a dilator in accordance with claim 1. Preferred embodiments thereof are subject-matter of dependent claims.

Further, a part of the front end side of the shaft may have a tapered shape having an outer diameter decreasing toward the front end side.

Further, pitches of the spirally-arranged protruding portion may be constant at the part of the front end side of the shaft having a tapered shape.

Further, the shaft may include a first coil having a wire wound around into a hollow shape.

Further, if the shaft includes the first coil having a wire wound around into a hollow shape, and the spirally-arranged protruding portion includes the second coil having a wire wound around on the outer peripheral surface of the shaft, wires of the first coil and the second coil may be wound around in directions opposite to each other.

### ADVANTAGEOUS EFFECTS OF INVENTION

The present invention can provide a dilator capable of easily increasing the diameter of a hole formed on the wall of the digestive tract and the like.

### BRIEF DESCRIPTION OF DRAWINGS

Fig. 1 shows an overall view of a dilator according to a first embodiment of the present invention.
Fig. 2 shows a front-end side portion of a dilator according to a second embodiment.
Fig. 3 shows a front-end side portion of a dilator according to a third embodiment.

### DESCRIPTION OF EMBODIMENTS

Below, the embodiments of the present invention will be described with reference to the figures. It is noted that the dimensions of dilators shown in the figures are merely provided to facilitate understanding of the embodiments, but do not necessarily correspond to the actual dimensions.

### First embodiment

A first embodiment of the present invention will be described with reference to the figures.

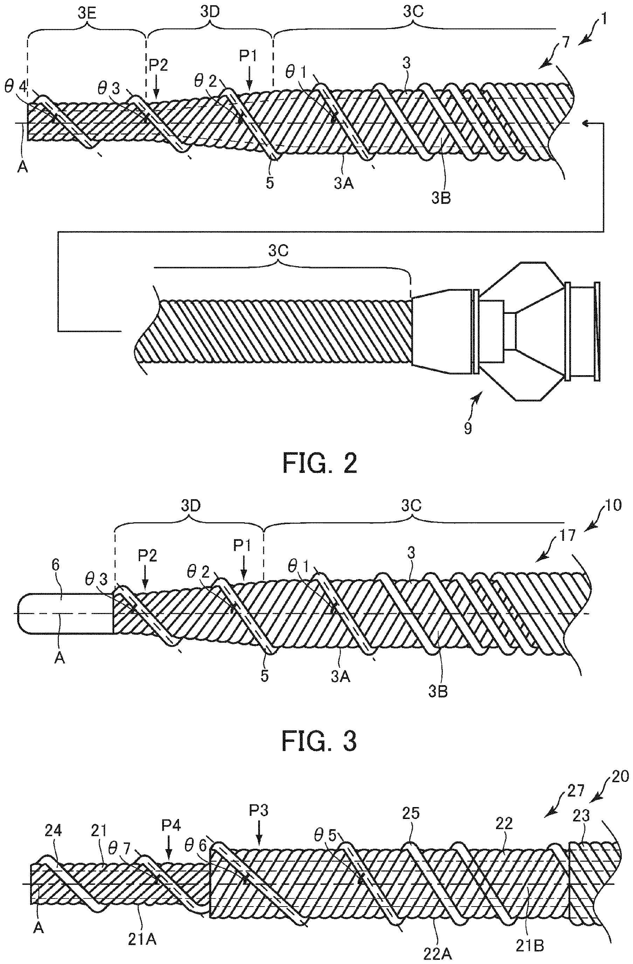

Fig. 1 shows an overall view of a dilator 1 according to the first embodiment of the present invention.

In Fig. 1, the left side in the figure corresponds to the front end side (the distal side) which is to be inserted into the body, and the right side corresponds to the base end side (the hand side, the proximal side) which is to be operated by an operator such as a surgeon.

In Fig. 1, a dilator 1 includes a multilayer body 7 including a first coil 3 including a plurality of metal wires wound around into a hollow shape and a second coil 5 including a single metal wire wound around on an outer peripheral surface 3A of the first coil 3 in a direction (clockwise, facing to the front end) opposite to the first coil 3 (counterclockwise, facing to the front end); and a connector 9 having a hollow shape and connected to a base end of the multilayer body 7.

Wires of the first coil 3 and the second coil 5 are, for example, metal wires of stainless steel, a superelastic alloy such as nickel-titanium, and the like, or resin wires.

The first coil 3 is configured such that metal wires of, for example, 10 stainless steel wires are wound around. The first coil 3 has a hollow shape in which an inner cavity 3B is formed penetratingly extending from a base end to a front end. The first coil 3 has a body portion 3C, a tapered portion 3D, and a front end portion 3E. The first coil 3 corresponds to a shaft.

The body portion 3C is located in the base end side of the dilator 1, and the connector 9 is connected to a base end thereof. Further, the body portion 3C has a substantially constant outer diameter from the base end thereof through a front end.

The tapered portion 3D is located in the front end side of the body portion 3C, and extends to the front end side from the front end of the body portion 3C, and is configured so as to have an outer diameter becoming smaller toward the front end side. The tapered portion 3D corresponds to a part of the front end side of the shaft.

The front end portion 3E is located in the front end side of the tapered portion 3D, and extends to the front end side from a front end of the tapered portion 3D. Further, the front end portion 3E has a substantially constant outer diameter from a base end thereof through a front end. As described above, the first coil 3 which corresponds to a shaft has a hollow shape having an outer diameter of a front end smaller than that of a base end.

The second coil 5 includes, for example, a single metal wire wound around on the outer peripheral surface 3A of the first coil 3 in a direction (clockwise, facing to the front end) opposite to the first coil 3 (counterclockwise, facing to the front end). Here, the metal wire is wound around closely at the base end side, and wound around with gap between adjacent windings at the front end side of the body portion 3C, the tapered portion 3D, and the front end portion 3E. A portion of the second coil 5 wound around with gap provides a spirally-arranged protruding portion protruding outwardly (outermost portion of the dilator 1, the outermost surface) on the outer peripheral surface 3A of the first coil 3. The above spirally-arranged protruding portion has gaps between adjacent portions (adjacent portions of a metal wire) along an axis A of the first coil 3. The screw action of the above spirally-arranged protruding portion enables the dilator 1 to be advanced even by a rotational operation of the dilator 1.

Further, the second coil 5 at a portion spaced to each other along the axis A is configured so that inclination angles to the axis A of the first coil 3 become gradually smaller toward the tapered portion 3D and the front end portion 3E from the body portion 3C. In the present embodiment, Θ1 to θ4 in Fig. 1 is such that Θ1 > θ2 > Θ3 > θ4. That is, the inclination angle at a second position P2 is configured so as to be smaller than that at a first position P1 along the axis A. It is noted that the second position P2 is located in the front end side relative to the first position P1, and corresponds to a position having an outer diameter smaller than that at the first position P1.

Further, with regard to the metal wire of the second coil 5, the amount of gap between adjacent portions of the metal wire is gradually decreased toward the base end side thereof at the body portion 3C. This configuration enables the stiffness of the dilator 1 (the multilayer body 7) along the axis direction to be gradually changed so that the dilator 1 can easily enter into an approach pathway even when the pathway meanders.

In the present embodiment and other embodiments described hereinafter, the length of a dilator is, for example, 2000 mm, preferably 1600 mm to 2500 mm, and the length of the front end portion 3E is, for example, 10 mm, preferably 0 to 100 mm, and the length of the tapered portion 3D is, for example, 30 mm, preferably 5 to 100 mm. The inner diameter of the front end of the first coil 3 is, for example, 0.7 mm, preferably 0.4 to 1.0 mm, and the inner diameter of the base end of the first coil 3 is, for example, 1.5 mm, preferably 1.0 to 3.0 mm. The outer diameter of the front end of the second coil 5 is, for example, 1.84 mm, preferably 0.8 to 3.0 mm, and the outer diameter of the base end of the second coil 5 is, for example, 2.64 mm, preferably 1.4 mm to 5.0 mm. Further, the diameters of the metal wires of the first coil 3 are, for example, 0.21 mm, preferably 0.1 to 0.5 mm, and the diameter of the metal wire of the second coil 5 is, for example, 0.36 mm, preferably 0.1 to 0.5 mm.

Further, an inclination angle (θ1) of the second coil 5 (the spirally-arranged protruding portion) at the body portion 3C is 70°, and an inclination angle (θ4) of the second coil 5 (the spirally-arranged protruding portion) at the front end portion 3E is 60°, and the ratio of them (Θ1/Θ4) is 1.17. It is noted that the inclination angle (θ1) of the second coil 5 (the spirally-arranged protruding portion) at the body portion 3C is preferably 14 to 87°, and the inclination angle (θ1) of the second coil 5 (the spirally-arranged protruding portion) at the front end portion 3E is preferably 7 to 82°, and the ratio of them (Θ1/Θ4) preferably ranges from 1.06 to 12.4.

The connector 9 as a grip portion is a portion through which an operator pushes the dilator into the body, and/or performs a rotational operation. A front end of the connector 9 is connected to the base end of the first coil 3 and the base end of the second coil 5. The connector 9 is made of a resin, and has a hollow shape having an inner cavity in communication with the inner cavity 3B of the first coil 3.

In the dilator 1 according to the present embodiment, the spirally-arranged protruding portion (the second coil 5) protruding outwardly is provided on the outer peripheral surface 3A of the first coil 3 which corresponds to a shaft, and has gaps between adjacent portions along the axis A of the first coil 3. This configuration enables the dilator to be advanced not only by a conventional pushing operation, but also by a rotational operation of the spirally-arranged protruding portion.

Further, for the spirally-arranged protruding portion (the second coil 5) of the dilator 1 according to the present embodiment, the inclination angle at the second position P2 is configured to be smaller than that at the first position P1 along the axis A. It is noted that the second position P2 is located in the front end side relative to the first position P1, and corresponds to a position in which the outer diameter is smaller than that of the first position P1. Therefore, the inclination angles of the spirally-arranged protruding portion are larger at a portion where the outer diameter of the first coil 3 which corresponds to a shaft is relatively large. This enables the spirally-arranged protruding portion to be firmly caught on a target object (for example, the digestive tract such as stomach, and liver). This, in turn, can prevent free rotation of the dilator 1 at the portion where the outer diameter of the first coil 3 which corresponds to a shaft is relatively large.

Further, the first coil 3 as a shaft having the tapered portion 3D at a part of the front end side can lead to smooth expansion of a hole.

Further, a shaft comprised of the first coil 3 including a plurality of metal wires wound around into a hollow shape can improve the flexibility of the shaft and the transmissibility of torque. Further, a spirally-arranged protruding portion comprised of the second coil 5 including a single metal wire wound around on the outer peripheral surface 3A of the first coil 3 can be easily formed, and can ensure the flexibility of the front end of the dilator 1 by virtue of the elasticity of the second coil 5, and can improve the torquability. Further, wires of the first coil 3 and the second coil 5 are wound around in directions opposite to each other. Therefore, even when the dilator 1 is rotated in a direction to open the first coil 3, a force is applied in a direction to close the second coil 5 to prevent opening of the first coil 3. This allows a force applied to the connector 9 of the dilator 1 to be transmitted to the front end side.

Next, an example of operating modes of the above dilator will be described.

First, a target object is punctured with an introducer needle to open a hole. Subsequently, a guide wire is inserted into an inner cavity of the introducer needle, and then the introducer needle is withdrawn.

Next, the base end of the guide wire is inserted into an inner cavity of the above dilator, and then the dilator is inserted. Subsequently, the dilator is pushed in while rotating a shaft to expand the hole at a punctured portion. During this, the tapered portion moves forward by virtue of the screw action of the spirally-arranged protruding portion by a rotational operation of the shaft and others, enabling the tapered portion to smoothly expand the hole.

### Second embodiment

Fig. 2 shows a front-end side portion of a dilator 10 according to a second embodiment.

In Fig. 2, the left side in the figure corresponds to the front end side (the distal side) which is to be inserted into the body, and the right side corresponds to the base end side (the hand side, the proximal side) which is to be operated by an operator such as a surgeon.

It is noted that the dilator 10 according to the present embodiment basically has the same structure as the dilator 1 according to the first embodiment. Therefore, the same number is given to the same member, and detailed description will be omitted.

In Fig. 2, a dilator 10 includes a multilayer body 17 including the first coil 3 having a plurality of metal wires wound around into a hollow shape and the second coils 5 having a single metal wire wound around on the outer peripheral surface 3A of the first coil 3 in a direction (clockwise, facing to the front end) opposite to the first coil 3 (counterclockwise, facing to the front end); and a connector 9 having a hollow shape and connected to a base end of the multilayer body 17 (see Fig. 1). However, the dilator 10 differs from the dilator 1 in that the dilator 10 has a forefront portion 6 instead of the front end portion 3E of the first coil 3 of the dilator 1. According to the present embodiment, the first coil 3 having the forefront portion 6 provided at the front end corresponds to a shaft.

The forefront portion 6 is formed by casting a solder material (a silver-tin solder material, a gold-tin solder material, and the like) into the front end of the hollow coil body 3, and has a substantially tubular hollow shape. Further, the forefront portion 6 has a flat surface while the front end of the multilayer body 7 has an uneven surface.

In the present embodiment, the second coil 5 at a portion spaced to each other along the axis A is also configured so that inclination angles to the axis A of the first coil 3 become gradually smaller toward the tapered portion 3D and the front end portion 3E from the body portion 3C. In the present embodiment, Θ1 to Θ3 in Fig. 2 is such that Θ1 > θ2 > Θ3 as in the first embodiment. That is, the inclination angle at the second position P2 is configured so as to be smaller than that at the first position P1 along the axis A. It is noted that the second position P2 is located in the front end side relative to the first position P1, and corresponds to a position having an outer diameter smaller than that of the first position P1.

The dilator 10 having this configuration can produce similar effects as the dilator 1 according to the first embodiment. Further, the forefront portion 6 having a flat surface is connected to the front end of the multilayer body 17. This configuration can further improve insertability into a punctured portion by first pressing the dilator against the punctured portion, and then pushing and rotating the dilator thereinto.

### Third embodiment

Fig. 3 shows a front-end side portion of a dilator 20 according to a third embodiment.

In Fig. 3, the left side in the figure corresponds to the front end side (the distal side) which is to be inserted into the body, and the right side corresponds to the base end side (the hand side, the proximal side) which is to be operated by an operator such as a surgeon.

In Fig. 3, the dilator 20 includes a multilayer body 27 including a hollow coil body 21, a coil body 22, a coil body 23, a coil body 24, and a coil body 25; and a connector 9 (Fig. 1) having a hollow shape and connected to a base end of the multilayer body 27.

The hollow coil body 21 is configured such that a plurality of metal wires (for example, 10 wires) are wound around into a tubular hollow shape. In Fig. 3, the dotted line (the innermost among the three dotted lines) represents the common inscribed line of the hollow coil body 21, and an inner cavity 21B is formed inside the common inscribed line of the hollow coil body 21.

A front end of the coil body 22 is located in the base end side relative to a front end of the hollow coil body 21, and the coil body 22 is configured such that a plurality of metal wires (for example, 16 wires) are wound around on an outer peripheral surface 21A of the hollow coil body 21 into a tubular hollow shape. The plurality of metal wires of the coil body 22 are wound around in the same direction (counterclockwise, facing to the front end) as the hollow coil body 21. In Fig. 3, the dotted line (the intermediate among the three dotted lines) represents the common inscribed line of the hollow coil body 22.

A front end of the coil body 23 is located at the base end side relative to the front end of the hollow coil body 22, and the coil body 23 is configured such that a plurality of metal wires (for example, 23 wires) are wound around on an outer peripheral surface 22A of the hollow coil body 22 into a tubular hollow shape. The plurality of metal wires of the coil body 23 are wound around in a direction (clockwise, facing to the front end) opposite to the coil body 22 (counterclockwise, facing to the front end). In Fig. 3, the dotted line (the outermost among the three dotted lines) represents the common inscribed line of the hollow coil body 23.

The coil body 22 is twistedly formed on the outer peripheral surface 21A of the hollow coil body 21, and the coil body 23 is twistedly formed on the outer peripheral surface 22A of the coil body 22. This means that the hollow coil body 21 and the coil body 22 which correspond to the shaft have a hollow shape having an outer diameter of a front end smaller than that of a base end.

The coil body 24 is configured such that a single element wire is wound around with gap between adjacent windings on the outer peripheral surface 21A of the coil body 21 in a direction (clockwise, facing to the front end) opposite to the coil body 21 (counterclockwise, facing to the front end). The coil body 25 is configured such that a single metal wire is wound around with gap between adjacent windings on the outer peripheral surface 22A of the coil body 22 in a direction (clockwise, facing to the front end) opposite to the coil body 22 (counterclockwise, facing to the front end).

In the present embodiment, the multilayer body 27 has a stepped and tubular hollow shape without the tapered portion.

It is noted that the hollow coil body 21 and the coil body 22 correspond to the shaft and the first coil, and the coil bodies 24 and 25 correspond to the spirally-arranged protruding portion and the second coil.

According to the present embodiment, in the hollow coil body 21, the coil body 22, and the coil body 23, each wire is wound around closely while in the coil body 24 and the coil body 25, each wire is wound around with gap between adjacent windings. The coil body 24 provides a spirally-arranged protruding portion protruding outwardly (outermost portion of the dilator 20, the outermost surface) on an outer peripheral surface 21L of the hollow coil body 21, and the coil body 25 provides a spirally-arranged protruding portion protruding outwardly (outermost portion of the dilator 20, the outermost surface) on an outer peripheral surface 22T of the coil body 22. The above spirally-arranged protruding portions have gaps between adjacent portions (adjacent portions of a metal element wire) along an axis A of the hollow coil body 21.

It is noted that in the present embodiment, wires of the hollow coil body 21, the coil body 22, the coil body 23, the coil body 24, and the coil body 25 may be metal wires made of stainless steel, a superelastic alloy such as nickel-titanium or the like. They shall not be limited to metal wires, but may be resin wires.

Further, the coil body 24 and the coil body 25 are configured so that inclination angles to the axis A of the hollow coil body 21 and the coil body 22 become gradually smaller from the base end side to the front end side (from the side of the coil body 22 to the side of the hollow coil body 21). In the present embodiment, θ5 to θ7 in Fig. 3 is such that θ5 > θ6 > θ7. That is, the inclination angle at a second position P4 is configured so as to be smaller than that at a first position P3 along the axis A. It is noted that the second position P4 is located at the front end side relative to the first position P3, and corresponds to a position having an outer diameter smaller than that of the first position P3.

In the dilator 20 according to the present embodiment, the spirally-arranged protruding portions (the coil bodies 24 and 25) are configured so that the inclination angles are smaller at the second position P4 than at the first position P3 along the axis A. It is noted that the second position P4 is located at the front end side relative to the first position P3, and corresponds to a position having an outer diameter smaller than that of the first position P3. Therefore, the inclination angles of the spirally-arranged protruding portion are larger at the coil body 22 which corresponds a portion where the outer diameter of the shaft is relatively large. This enables the spirally-arranged protruding portion to be firmly caught on a target object (for example, the digestive tract such as stomach, and liver). This, in turn, can prevent free rotation of the dilator 20 at coil body 22 which corresponds to a portion where the outer diameter of the shaft is relatively large.

Further, a shaft composed of the hollow coil body 21 and the coil body 22 (the first coil) each including a plurality of metal wires wound around into a hollow shape can improve the flexibility of the shaft and the transmissibility of torque via the shaft. Further, a spirally-arranged protruding portion composed of the coil body 24 (the second coil) including a single metal wire wound around on the outer peripheral surface 21A of the hollow coil body 21 and the coil body 25 (the second coil) wound around on the outer peripheral surface 22A of the coil body 22 can be easily formed, and can ensure the flexibility of the front end of the dilator 20 by virtue of the elasticity of the second coil, and can improve the torquability. Further, each wire of the hollow coil body 21 and the coil body 22 and each wire of the coil body 24 and the coil body 25 are wound around in directions opposite to each other. Therefore, even when the dilator 20 is rotated in a direction to open the hollow coil body 21 and the coil body 22, a force is applied in a direction to close the coil body 24 and the coil body 25 to prevent opening of the hollow coil body 21 and the coil body 22. This allows a force applied to the connector 9 of the dilator 20 to be transmitted to the front end side.

Hereinbefore, the embodiments of the present invention are described, but the present invention shall not be limited to these embodiments. Rather, various modifications may be made.

For example, the first coils 3 and 21 are each described as a hollow coil body including 10 wires in the aforementioned embodiments, but the number of wires shall not be limited to 10. The number may be one or more.

Further, the coil body 22 is described as a coil body including 16 wires in the aforementioned embodiments, but the number of wires shall not be limited to 16. The number may be one or more.

Further, the coil body 23 is described as a coil body including 23 wires in the aforementioned embodiments, but the number of wires shall not be limited to 23. The number may be one or more.

Further, the forefront portion 6 according to the second embodiment is described to be formed by casting a solder material into the front end of the multilayer body 17. However, the outer periphery of the second coil 5 and/or the first coil 3 in the vicinity of the front end portion of the multilayer body 17 may be ground to form the forefront portion 6 having a flat surface.

Furthermore, the forefront portion 6 according to the second embodiment is described to be fixed to the front end of the multilayer body 17, but the forefront portion may be fixed to the front end of the multilayer body 27 according to the third embodiment.

Further, the outer peripheries of the multilayer bodies 7, 17, and 27 according to the first to third embodiments may be coated with a resin(s).

Further, the second coils 5, 24, and 25 are configured so that the inclination angles are gradually decreased in the above embodiments, but the inclination angles are configured to be stepwisely decreased. Further, for the first positions P1, and P3 and the second positions P2, and P4 are not limited to the positions in the above embodiments, but the first and second positions may be located at any positions as long as the second position is located in the front end side relative to the first position, and has outer diameter smaller than that of the first position.

In the embodiments shown in Figs. 1 to 3, illustrated are dilators each including a shaft having no surface coating. However, the shaft may have various types of coating on the side of the surface thereof (including a portion between the shaft and the spirally-arranged protruding portion). Examples of the coating include, for example, a protective film on the surface of the shaft (representative example: a plating film), an underlying film for improving adhesiveness between the shaft and the spirally-arranged protruding portion, and the like.

Preferably, the spirally-arranged protruding portions according to the embodiments as shown in Figs. 1 to 3 are not configured to serve as a blade. The dilators according to the present embodiments are intended for expanding a hole pre-formed on a target object (for example, the wall of the digestive tract such as the patient's stomach). Therefore, if the spirally-arranged protruding portion serves as a blade, living body tissues at the inner surface of the hole may be damaged.

For this reason, the spirally-arranged protruding portion preferably does not have a sharp edge at an end portion in a radially outer side of the shaft on a cross-section. That is, the above end portion preferably has an area having a shape including an obtuse angle or a curve (for example, a curve constituting a part of a circle or an ellipse).

### REFERENCE SIGNS LIST

1, 10 Dilator
3 First coil
3A, 21A, 22A Outer peripheral surface
5 Second coil
9 Connector
21 Hollow coil body 2
22, 24, 25 Coil body
P1, P3 First position
P2, P4 Second position
Θ1, θ2, Θ3, θ4, θ5, θ6, θ7 Inclination angle

## Claims

1. A dilator (1; 10; 20) comprising:
a hollow shaft (3; 21, 22) having an outer diameter of a front end smaller than that of a base end; and
a grip portion (9) provided at the base end of the shaft (3; 21, 22), wherein,
a spirally-arranged protruding portion (5; 24, 25) protruding outwardly is provided on an outer peripheral surface of the shaft (3; 21, 22),
the spirally-arranged protruding portion (5; 24, 25) has gaps between adjacent portions along an axis (A) of the shaft (3; 21, 22),
the shaft (3; 21, 22) has a first position (P1; P3) and a second position (P2; P4) along the axis (A), the second position (P2; P4) being located at a front end side relative to the first position (P1; P3) and having an outer diameter smaller than that of the first position (P1; P3), and
the spirally-arranged protruding portion (5; 24, 25) has an inclination angle to the axis (A) at the second position (P2; P4) smaller than that at the first position (P1; P3), **characterized in that**
the spirally-arranged protruding portion (5; 24, 25) includes a coil having a wire wound around on the outer peripheral surface (3A; 21A, 22A) of the shaft (3; 21, 22).

2. The dilator (1; 10; 20) according to claim 1, wherein a part of the front end side of the shaft (3; 21, 22) has a tapered shape having an outer diameter decreasing toward the front end side.

3. The dilator (1; 10; 20) according to claim 2, wherein pitches of the spirally-arranged protruding portion are constant in the part of the front end side of the shaft having a tapered shape.

4. The dilator (1; 10; 20) according to any one of claims 1 to 3, wherein
the shaft (3; 21, 22) includes a first coil having a wire wound around into a hollow shape, and
the coil of the spirally-arranged protruding portion (5; 24, 25) forms a second coil.

5. The dilator according to claim 4, wherein,
the wires of the first coil and the second coil are wound around in directions opposite to each other.

## Patentansprüche

1. Dilatator (1; 10; 20) aufweisend:
einen hohlen Schaft (3; 21, 22), bei dem ein Außendurchmesser eines vorderen Endes kleiner ist als der eines hinteren Endes; und
einen Griffabschnitt (9), der am hinteren Endes des Schafts (3; 21, 22) vorgesehen ist, wobei
ein spiralförmig angeordneter vorstehender Abschnitt (5; 24, 25), der nach außen vorsteht, an einer Außenfläche des Schafts (3; 21, 22) vorgesehen ist,
der spiralförmig angeordnete vorstehende Abschnitt (5; 24, 25) Lücken zwischen benachbarten Abschnitten entlang einer Achse (A) des Schafts (3; 21, 22) aufweist,
der Schaft (3; 21, 22) eine erste Position (P1; P3) und eine zweite Position (P2; P4) entlang der Achse (A) hat, wobei die zweite Position (P2; P4) an einer vorderen Endseite relativ zu der ersten Position (P1; P3) angeordnet ist und einen Außendurchmesser hat, der kleiner ist als der der ersten Position (P1; P3), und
der spiralförmig angeordnete vorstehende Abschnitt (5; 24, 25) an der zweiten Position (P2; P4) einen Neigungswinkel zur Achse (A) aufweist, der kleiner ist als derjenige an der ersten Position (P1; P3), **dadurch gekennzeichnet, dass**
der spiralförmig angeordnete vorstehende Abschnitt (5; 24, 25) eine Spule mit einem auf der Außenumfangsfläche (3A; 21A, 22A) des Schafts (3; 21, 22) aufgewickelten Draht umfasst.

2. Dilatator (1; 10; 20) nach Anspruch 1, wobei ein Teil der vorderen Endseite des Schafts (3; 21, 22) eine konische Form mit einem zur vorderen Endseite hin abnehmenden Außendurchmesser aufweist.

3. Dilatator (1; 10; 20) nach Anspruch 2, wobei die Steigungen des spiralförmig angeordneten vorstehenden Abschnitts in dem Teil der vorderen Endseite des Schafts, der eine konische Form aufweist, konstant sind.

4. Dilatator (1; 10; 20) nach einem der Ansprüche 1 bis 3, wobei
der Schaft (3; 21, 22) eine erste Spule mit einem in eine Hohlform gewickelten Draht aufweist, und
die Spule des spiralförmig angeordneten vorstehenden Abschnitts (5; 24, 25) eine zweite Spule bildet.

5. Dilatator nach Anspruch 4, wobei,
die Drähte der ersten Spule und der zweiten Spule in zueinander entgegengesetzten Richtungen gewickelt sind.

## Revendications

1. Dilatateur (1 ; 10 ; 20) comprenant :
un arbre creux (3 ; 21, 22) présentant un diamètre extérieur d'une extrémité avant inférieur à celui d'une extrémité de base ; et
une partie de préhension (9) disposée sur l'extrémité de base de l'arbre (3 ; 21, 22), dans lequel,
une partie saillante agencée en spirale (5 ; 24, 25) faisant saillie vers l'extérieur est disposée sur une surface périphérique extérieure de l'arbre (3 ; 21, 22),
la partie saillante agencée en spirale (5 ; 24, 25) présente des espaces entre des parties adjacentes le long d'un axe (A) de l'arbre (3 ; 21, 22),
l'arbre (3 ; 21, 22) présente une première position (P1 ; P3) et une seconde position (P2 ; P4) le long de l'axe (A), la seconde position (P2 ; P4) étant située au niveau d'un côté extrémité avant par rapport à la première position (P1; P3) et présentant un diamètre extérieur inférieur à celui de la première position (P1 ; P3), et
la partie saillante agencée en spirale (5 ; 24, 25) présente un angle d'inclinaison par rapport à l'axe (A) dans la seconde position (P2 ; P4) inférieur à celui dans la première position (P1 ; P3), **caractérisé en ce que**
la partie saillante agencée en spirale (5 ; 24, 25) inclut un enroulement présentant un fil enroulé sur la surface périphérique extérieure (3A ; 21A, 22A) de l'arbre (3 ; 21, 22).

2. Dilatateur (1; 10; 20) selon la revendication 1, dans lequel une partie du côté extrémité avant de l'arbre (3 ; 21, 22) présente une forme effilée présentant un diamètre extérieur diminuant vers le côté extrémité avant.

3. Dilatateur (1 ; 10 ; 20) selon la revendication 2, dans lequel des pas de la partie saillante agencée en spirale sont constants dans la partie du côté extrémité avant de l'arbre présentant une forme effilée.

4. Dilatateur (1 ; 10 ; 20) selon l'une quelconque des revendications 1 à 3, dans lequel
l'arbre (3 ; 21, 22) inclut un premier enroulement présentant un fil enroulé en une forme creuse, et
l'enroulement de la partie saillante agencée en spirale (5 ; 24, 25) forme un second enroulement.

5. Dilatateur selon la revendication 4, dans lequel,
les fils du premier enroulement et du second enroulement sont enroulés dans des directions opposées l'une à l'autre.
